# EUROPEAN PATENT SPECIFICATION

(11) **EP 1 343 826 B1**
(45) Date of publication and mention of the grant of the patent: **10.08.2005**
(21) Application number: 00981558.0
(22) Date of filing: 27.11.2000
(51) Int. Cl.: C08B 37/00, A61K 35/78

(54) **POLYSACCHARIDE COMPOUND HAVING IMMUNE STIMULATING ACTIVITY**
POLYSACCHARIDE MIT IMMUNOSTIMULIERENDER AKTIVITÄT
COMPOSE POLYSACCHARIDE PRESENTANT UNE ACTIVITE IMMUNO-STIMULANTE

(43) Date of publication of application: 17.09.2003
(73) Proprietor: Bomsund Grupo Asesor, S.L., 08008 Barcelona (ES)
(72) Inventor: FRIAS PENA, José Manuel, E-08008 Barcelona (ES)
(74) Representative: Carpintero Lopez, Francisco
(86) International application number: PCT/IB2000/001946
(87) International publication number: WO 2002/042335

(56) References cited:
- US-A- 4 857 512
- DATABASE WPI Week 200026 Derwent Publications Ltd., London, GB; AN 2000-301507 XP002177523 BOGDARIN YU A ET AL.: "Drug" & RU 2 129 433 C (NIZHEGOLD CHILD GASTROENTEROLOGY INST), 27 April 1999 (1999-04-27)
- VARLJEN J ET AL: "STRUCTURAL ANALYSIS OF A RHAMNOARABINOGALACTAN AND ARABINOGALACTANSWITH IMMUNO-STIMULATING ACTIVITY FROM CALENDULA OFFICINALIS" PHYTOCHEMISTRY, PERGAMON PRESS, GB, vol. 28, no. 9, 1989, pages 2379-2383, XP001025124 ISSN: 0031-9422

## Description

### FIELD OF THE INVENTION

The present invention relates to a polysaccharide compound of formula I which present immune stimulating activity, to a process for obtaining it, to its use in the treatment of immune suppressant diseases and to pharmaceutical compositions containing it.

### BACKGROUD OF THE INVENTION

The immune system can be defined as the collection of molecules, cells and organs whose complex interactions form an emergent system which is generally able to protect an individual both from outside invaders and its own altered cells.

The immune system can be separated into two functionally distinct parts: those elements which are innate and those which are acquired. Innate immunity refers to immune elements which are non-specific and non-adaptive. They are able to distinguish foreign tissues/organisms but are unable to recognise a particular invader. They can be best divided in barriers (skin and mucosa), non-specific chemical agents (enzymes present in mucusal secretions, quinines and histamines) and non-specific effector cells (macrophages). Acquired immunity refers to elements which are specific and adaptive. They are able to distinguish foreign cells from self, and can distinguish one foreign antigen from another. Acquired immunity has memory. This allows immunisation and resistance to reinfection with the same microorganism. The cells which are responsible for the acquired immunity are lymphocytes, of which there are two subclasses: B and T-cells.

The acquired immunity can be attained either by natural infection or vaccination (in an active way) or by administration of immune cells (in a passive way). While the first way presents long lasting effects and can be even permanent, the later is not long lasting.

Patients presenting immune-suppressant diseases are treated with immune stimulants in order to activate their immune system. Different immune stimulants are known from US 4801578, US 5417979, WO 9851319. Polysaccharide immune stimulants are known from DE 19817177 and EP 0 225 496. These patents disclose polysaccharide compounds having immune stimulating activity and their obtention from plant cell cultives. As plants *Echinacea purpurea, Echinacea angustifolia* and *Calendula officinalis* are employed.

However, none of these immune stimulating compounds present a satisfactory activity for the immune-suppressant diseases. There is, thus, a need in the art for alternative immune stimulators presenting an improved and wide activity.

### SUMMARY OF THE INVENTION

The present invention provides a polysaccharide compound of formula I which presents immune stimulating activity, a process for obtaining it, its use in the treatment of immune suppressant diseases and pharmaceutical compositions containing it.

An aspect of the invention relates to polysaccharide compounds of formula I 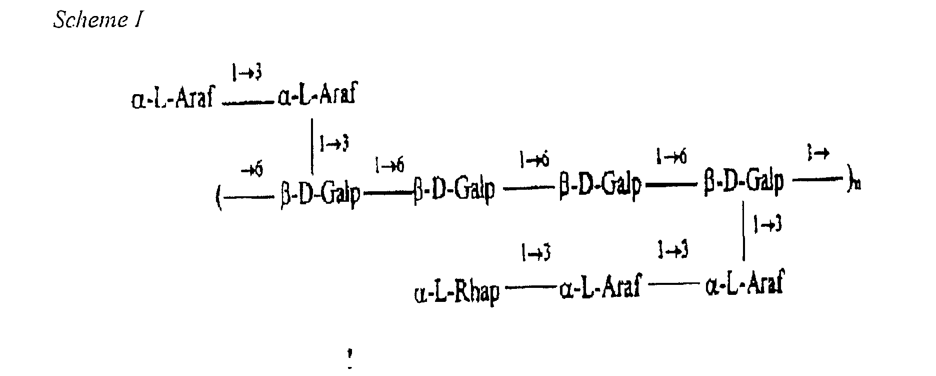 wherein n = 7 to 8.

A second aspect of the invention relates to the method for obtaining the polysaccharide compound of formula I. This method comprises firstly, the obtention of an aqueous extract from the plant *Calendula officinalis* according to a method disclosed in a copending application of ours and, secondly, the isolation of the polysaccharide by a employing a combination of separation techniques guided by bioassay.

A further aspect of the invention relates to the use of the present polysaccharide compound of formula I as therapeutic agent in the treatment of immune suppressant diseases such as cancer, tuberculosis, influenza, common cold, allergies, lupus erythematosus, psoriasis and AIDS.

A further aspect of the invention relates to pharmaceutical compositions comprising the polysaccharide compound of formula I.

The present application includes the present figures and tables:
Figure 1 is an schema of the isolation of PF2.
Figure 2 is an schema of the isolation of PF2R.
Figure 3 is the ¹H-RMN spectrum of PF2RS8A.
Figure 4 is the ¹³C-NMR spectrum of PF2RS8A.
Figure 5 is the TLC of the hydrolysis products of PF2RS8A with TFA and other monosugars.
Figure 6 is an schema of the isolation of PF2RS8B2, i.e., the polysaccharide of formula I.
Figure 7 is the HPLC profile of PF2RS8B2 under evaporative light scattering detector.
Figure 8 is the HPLC profile of PF2RS8B2 under photodiode UV detector.
Figure 9 is the ¹H-RMN spectrum of PF2RS8B2.
   Table 1 reflects the effect of samples PF2RS8A and PF2RS8B2.on Lymphocyte Transformation.
Figure 10 is the ¹³C-NNR spectrum of PF2RS8B2, i.e., the polysaccharide of formula I.
Figure 11 is the DEPT-135 spectrum of PF2RS8B2.
Figure 12 is the DEPT-90 spectrum of PF2RS8B2.
Figure 13 is the HMQC spectrum of PF2RS8B2.
Figure 14 is the ¹H-¹H COSY spectrum of PF2RS8B2.
Figure 15 is the ¹H-¹H COSY spectrum of PF2RS8B2.
Figure 16 is the HMQC spectrum of PF2RS8B2.
   Table 2 reflects the ¹³C-NMR spectrum of PF2RS8B2.
Figure 17 is the HMBC spectrum of PF2RS8B2.
Figure 18 is the HMBC spectrum of PF2RS8B2.
Figure 19 is the TLC of the hydrolysis products of PF2RS8B2 with TFA and other monosugars.
Figure 20 is the molecular weight measurement diagram.

### DETAILED DESCRIPTION OF THE INVENTION

As mentioned above, the first aspect of the invention relates to a polysaccharide compound of formula I: 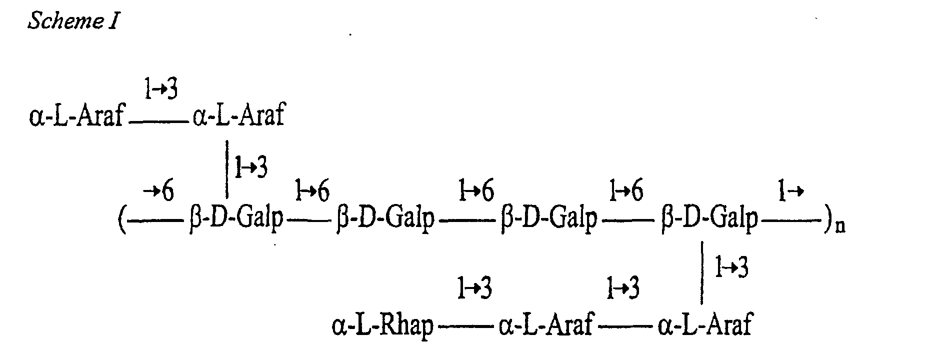 wherein n = 7 to 8
The configurational structure of the compound of formula I is: 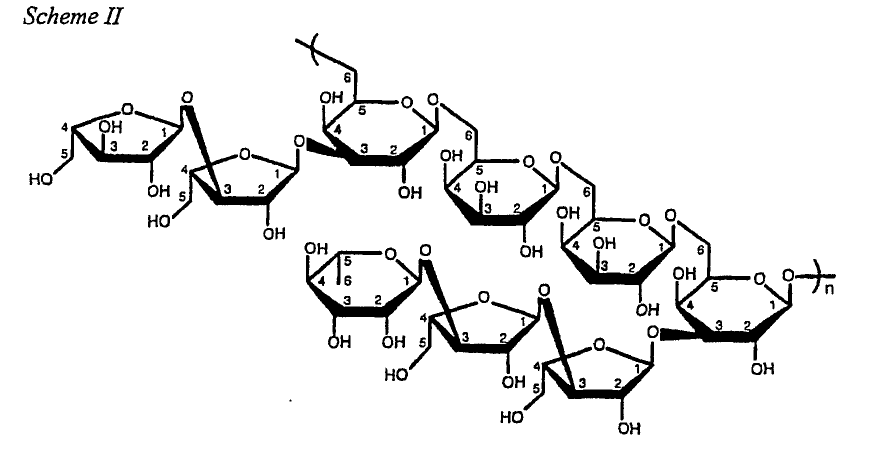 wherein n = 7 to 8

The polysaccharide compound present a mean molecular weight of about 10,000.

The second aspect of the invention relates to the method for obtaining the polysaccharide compound of formula I. This method comprises firstly, obtaining an aqueous extract from the plant *Calendula officinalis* according to a method disclosed in a copending patent application of ours entitled "process for preparing aqueous extracts of plants and extracts so obtained" and filed simultaneously with the present application of serial number PCT/IB 00/01947 (published as WO-A-02/41908 on 30 May 2002) and, secondly, the isolation of the polysaccharide by a combination of separation techniques guided by bioassay.

The aqueous extract of the *Calendula officinalis* is obtained by subjecting the flowers of this pant to the following process:
a) Decontamination of the flowers
b) Comminuting the flowers.
c) Treatment of the comminuted flowers with a laser radiation.
d) Suspension of the mixture obtained in step c) in water.
e) Maceration of the suspension obtained in step d).
f) Separation of the resulting liquid.

The decontamination (step a) is effected by washing the flowers of *Calendula oficinalis* with water. The amount of water employed in this step is not determinant, and can be varied depending on the contamination state of the plant. Although higher and lower temperatures are not discarded, the water temperature should be between 10 and 40 °C, preferably between 20 and 35°C, and most preferably 28°C. A washing tunnel can be employed to facilitate this step. Both the amount of water and the residence time of the plant in the washing tunnel is not determinant, and can therefore be varied depending on the contamination state of the plant. The washing step can be carried out several times, with a drying step in between. This drying step is preferably effected by placing the plant in the sun.

Once the flowers have been thoroughly decontaminated, they are comminuted (step b) by conventional methods such as a comminuting machine or even manually. Although higher and lower temperatures are not discarded, the temperature at which the flowers are comminuted should be between 10 and 40 °C.

The comminuted flowers are next subjected to a treatment with laser radiation (step c). As source of the laser radiation, a red linear laser diode with a capability of harmonic generation in wavelengths within the range of 150 to 810 nm is preferably employed. The wavelength of the laser radiation is more preferably of 200 to 400 nm and most preferably of 250 nm. The power of the laser radiation is preferably of 1 to 60 watts, more preferably of 10 to 30 watts and most preferably of 20 watts. The spot is preferably of 1 to 6 mm, more preferably of 2 to 5 mm and most preferably of 4 mm of diameter. The comminuted plant is exposed to the laser radiation so that the whole or most of the mixture is irradiated. This is achieved either by displacing manually the laser generator through the comminuted plant, or by passing the comminuted matter on a conveyor belt through a set of several laser generators. Preferably each kilogram of the comminuted matter is treated with the laser radiation for a period of 3 to 10 minutes, more preferably for a period of 5 minutes. Although higher and lower temperatures are not discarded, the temperature at which the comminuted plant is treated with the laser radiation should be between 10 and 40 °C.

The laser treated matter is next suspended in water (step d). Any commercial mineral water can be employed in this step. The suspension is effected so that 50 to 300, preferably 100 to 250, grams of the laser treated matter are present per litre of water. Although higher and lower temperatures are not discarded, the temperature at which the comminuted plant is suspended in water should be between 10 and 40 °C.

The suspension is then kept for a period of between 5 to 20 days, preferably of 7 to 15 days, at a temperature of 2 to 10 °C, preferably of 4 to 8°C, so that maceration of the mixture takes place (step e).

Finally, after the maceration step, a separation of the liquid phase from the solid phase is effected (step f). The solids can be pressed to facilitate the separation. The separation can be achieved by decantation alone or, preferably, by decantation followed by filtration. The filtration is preferably effected under pressure. Most preferably three consecutive press-filtrations are effected with filters of 5 µm, 1 µm and 0.22 µm. Although higher and lower temperatures are not discarded, the temperature at which the separation is effected should be between 10 and 40 °C.

An aqueous extract of an ochre colour is finally obtained.

The extract thus obtained is next subjected to an isolation process which comprises precipitation with methanol, centrifugation and chromatographic separation guided by bioassay. The bioassay employed were performed in vitro by adding the samples to lymphocytes isolated from mice according to the literature reference *Max, W. et al., Journal of Natural Products, vol. 54, no. 6, pp. 1531-1542 (1991).* The incorporation of thymidine, which means replication of DNA, was monitored. This incorporation is indicative both of an increase in lymphocyte number and an increase in lymphocyte activity. These separation techniques employed include repeated precipitation with ethanol, centrifugation, dialysis and/or column chromatography.

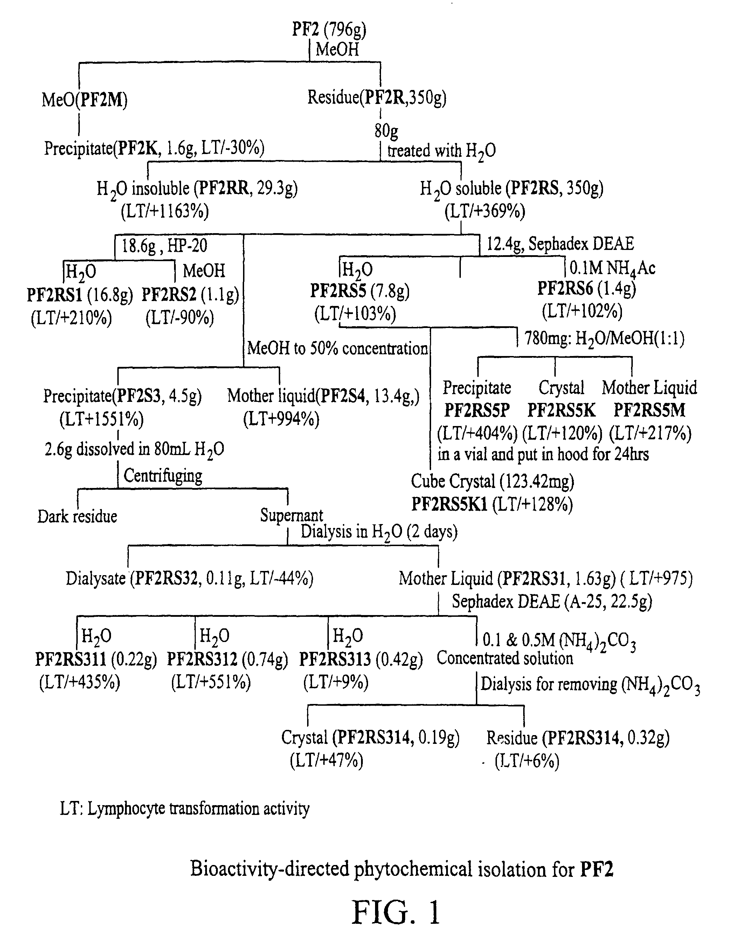

Thus, 112 litres of the aqueous extract previously obtained were lyophilised to yield 800 g of a tan coloured powder ("PF2"). This powder was fractionated into a MeOH-soluble and a MeOH-insoluble residue (PF2R, 350 g), which exhibit lymphocyte transformation activity. A portion (80 g) of this material was subjected to MeOH precipitation, centrifugation, dialysis and/or column chromatography on Sephadex DEAE led to the isolation of a number of crystalline materials, which were determined to be inactive inorganic salts (figure 1).

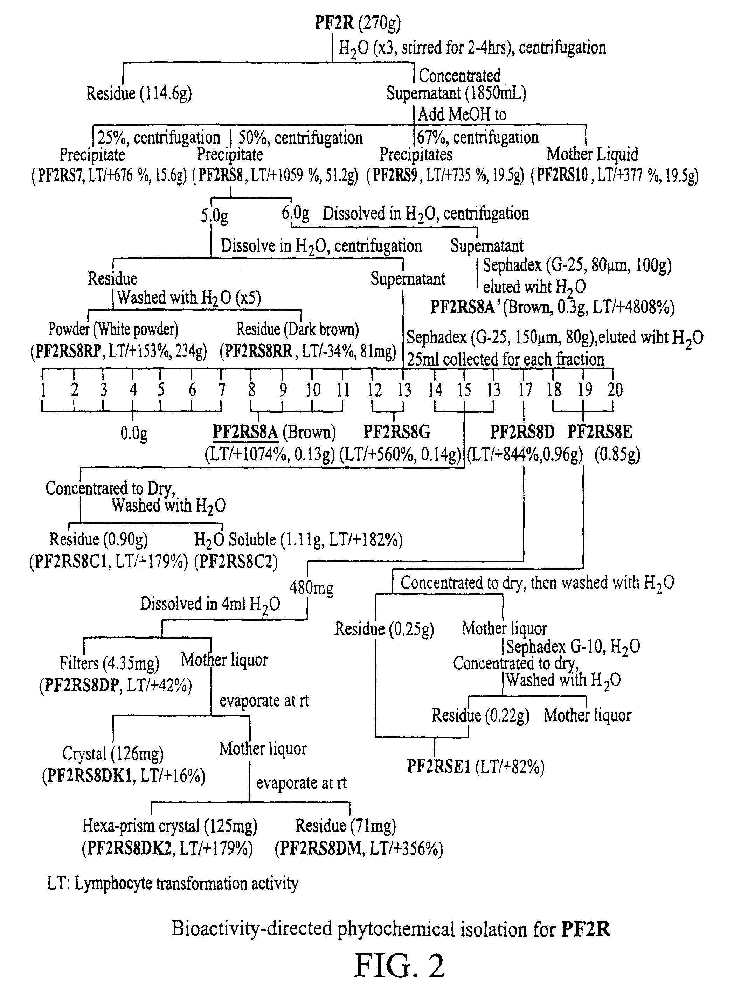

A portion (270 g) of PF2R which was subjected to precipitation with MeOH to final concentrations of 25, 50 and 67% led to active precipitates. The most active material was the precipitate obtained from the 50% MeOH solution (PF2RS8 51.2 g, LT= +1059 %). Work up of a 5 g portion of PF2RS8 solubilised in water, followed by centrifugation, and chromatographic separation of the supernatant on Sephadex G-25 led to the active polysaccharide enriched fraction designated as PF2RS8A (0.13 g, LT = +1074 %). Subsequently, an identical fraction designated as PF2RS8A' (0.3 g) was obtained from a second portion (6 g) of the 50 % MeOH precipitate. As indicated in figure 2, a number of other fractions and crystalline materials were isolated, however, none showed the level of LT activity of PF2RS8A.

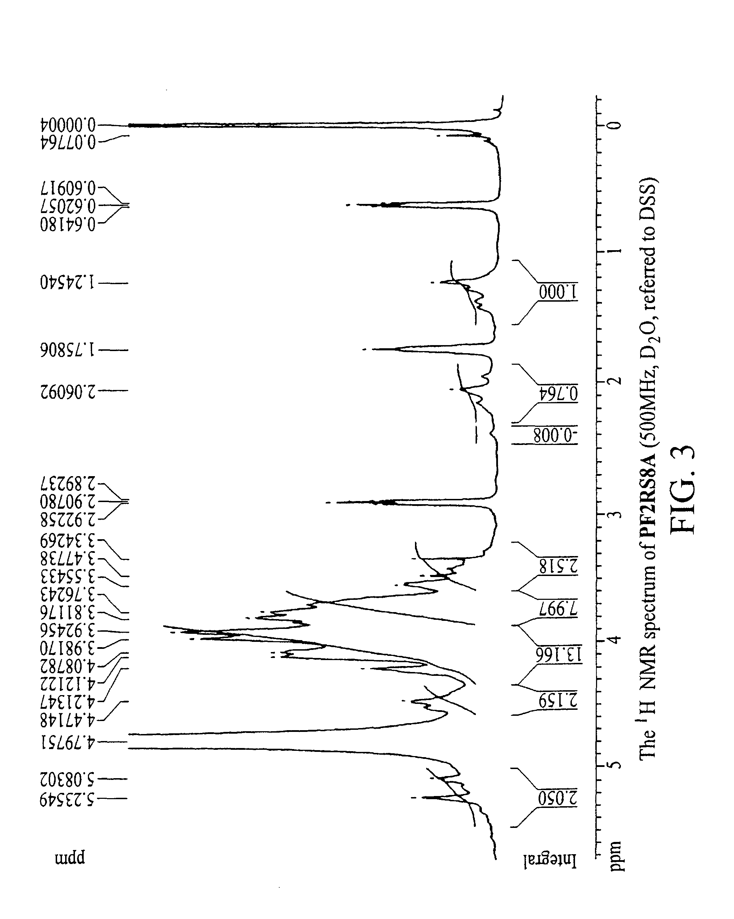

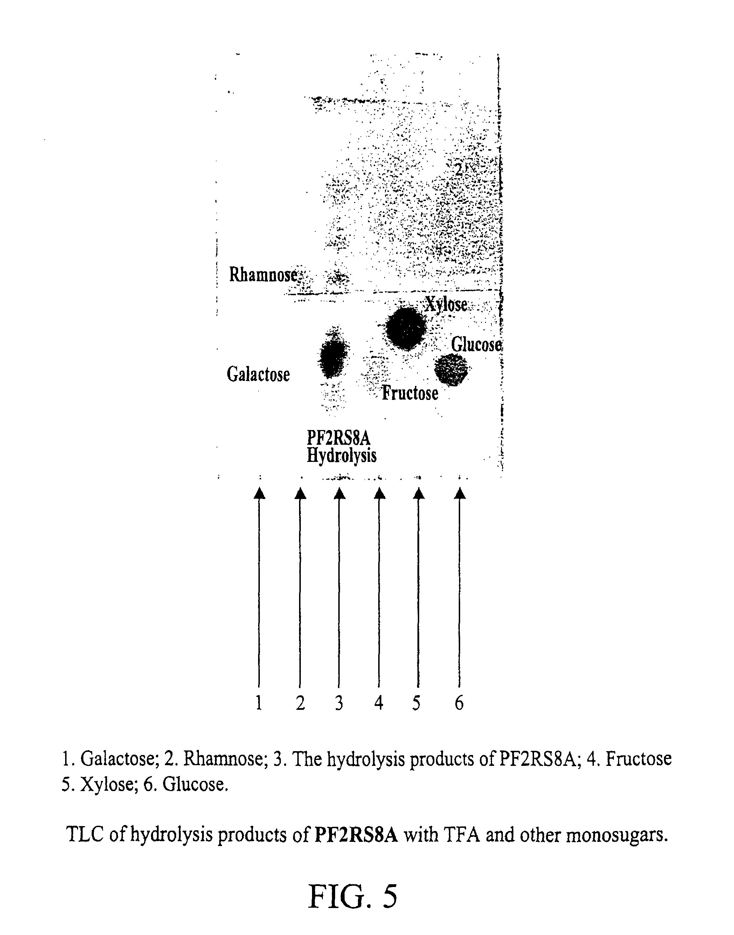

PF2RS8A was characterised by spectroscopic (¹H, ¹³C-NMR, and DEPT spectra) and chemical analysis (hydrolysis with TFA and analysis on silica gel TLC). See Figures 3, 4 and 5.

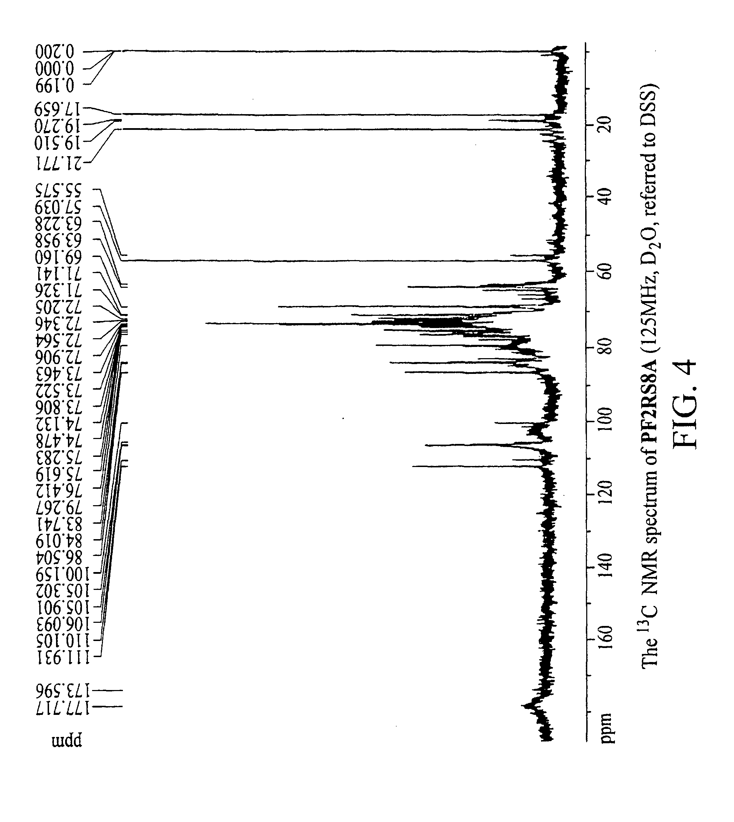

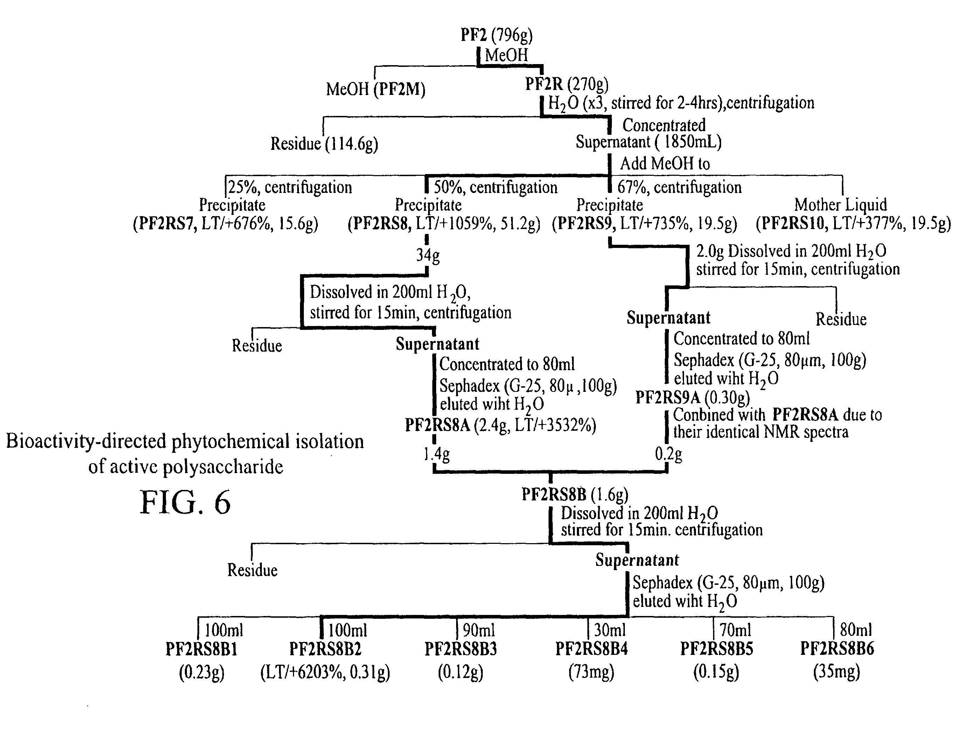

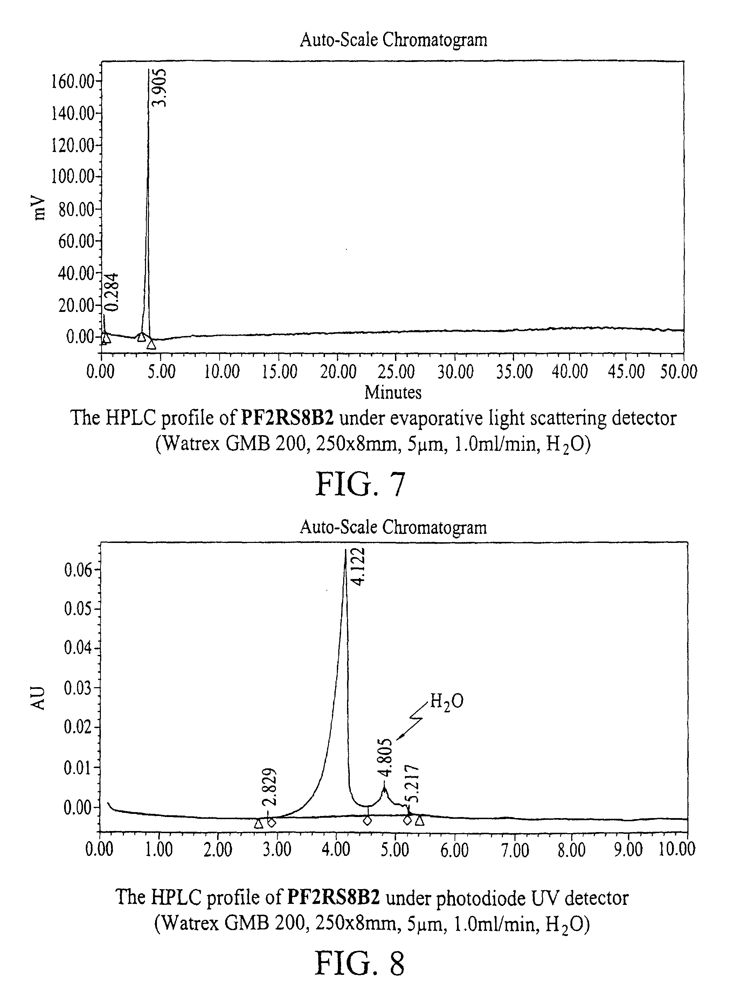

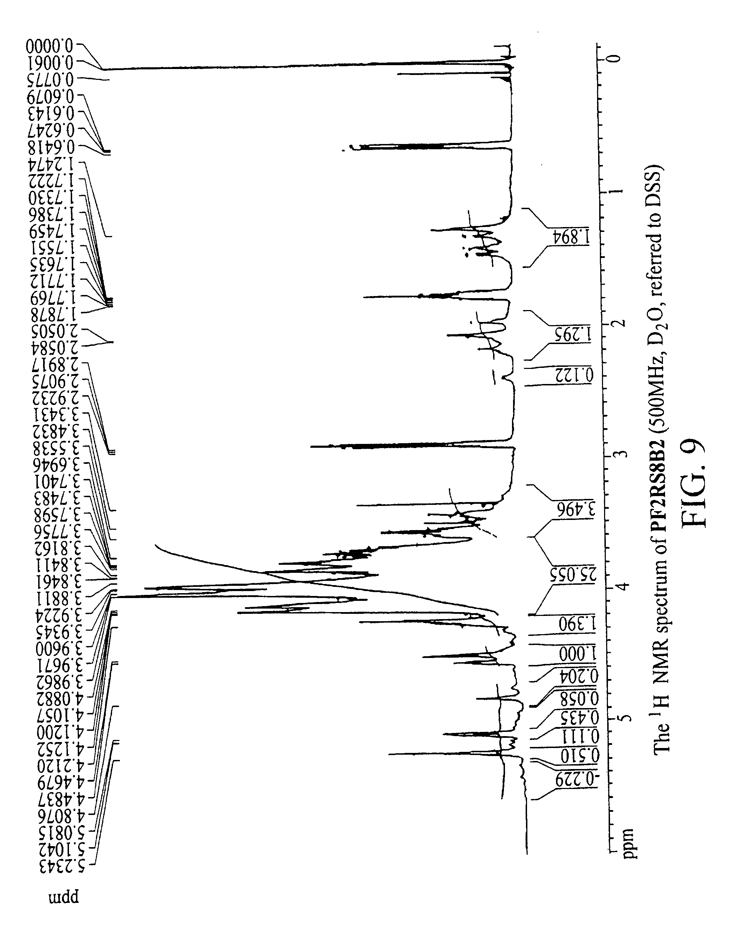

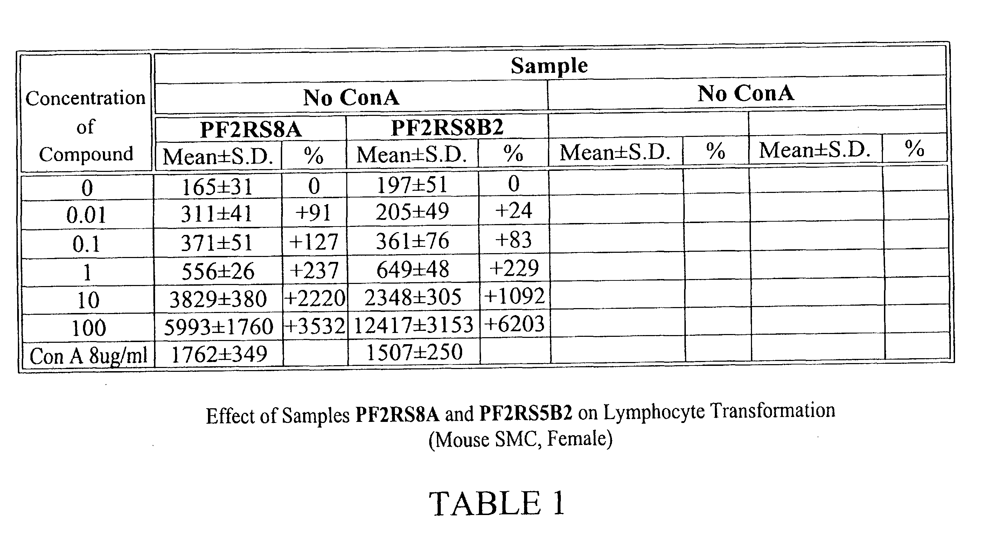

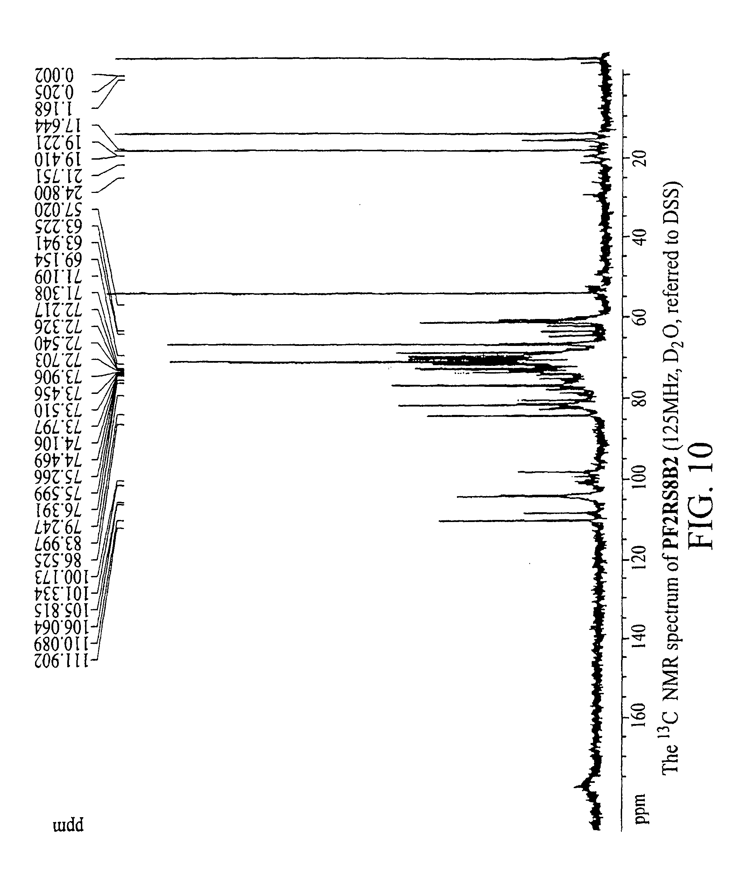

More active polysaccharide mixture PF2RS8A (1.4 g) was isolated from the remaining 50% MeOH precipitate (PF2RS8). At the same time, work-up of a portion (2.0 g) of the 67% MeOH insoluble fraction (PF2RS9; LT =+735%) led to the isolation of a polysaccharide mixture (PF2RS9A, 0.3g), which was showed by ¹H- NMR analysis to be identical as PF8RS8A. Thus, PF2RS9A (0.2 g) was combined with PF2RS8A (1.4 g), and the mixture, designated as "PF2S8B", was subjected to further separation with sephadex G-50 (20-80 mm) as showed in figure 6. Elution with water yielded six fractions (PF2RS8B1, 2, 3, 4, 5 and 6), which on HPLC analysis showed that only the major isolate PF2RS8B2 to be homogeneous using both evaporative light scattering and UV detection (figures 7 and 8). ¹H and ¹³C-NMR spectral analysis (figures 9 and 10) showed spectra of this isolate to be very similar to those obtained for PF2RS8A (figures 3 and 4), which implied that it is the major polysaccharide in the mixture. The fraction PF2RS8B2 consists therefore of the polysaccharide of formula I and water. The latter can be eliminated by methods known in the art.

Bioassay of the homogeneous isolate PF2RS8B2 and its parent mixture PF2RS8A in the same assay showed lymphocyte transformation (LT) activities of 6203% and 3532%, respectively (Table 1). The higher level of activity showed by the isolate implied that this polysaccharide is the major active constituent for the biological effect of the present extract.

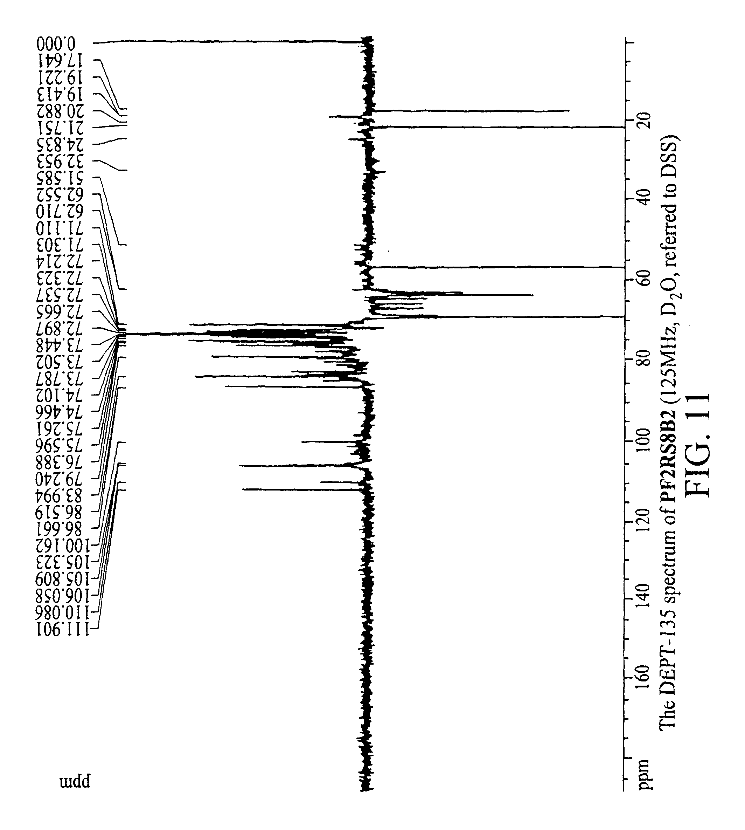

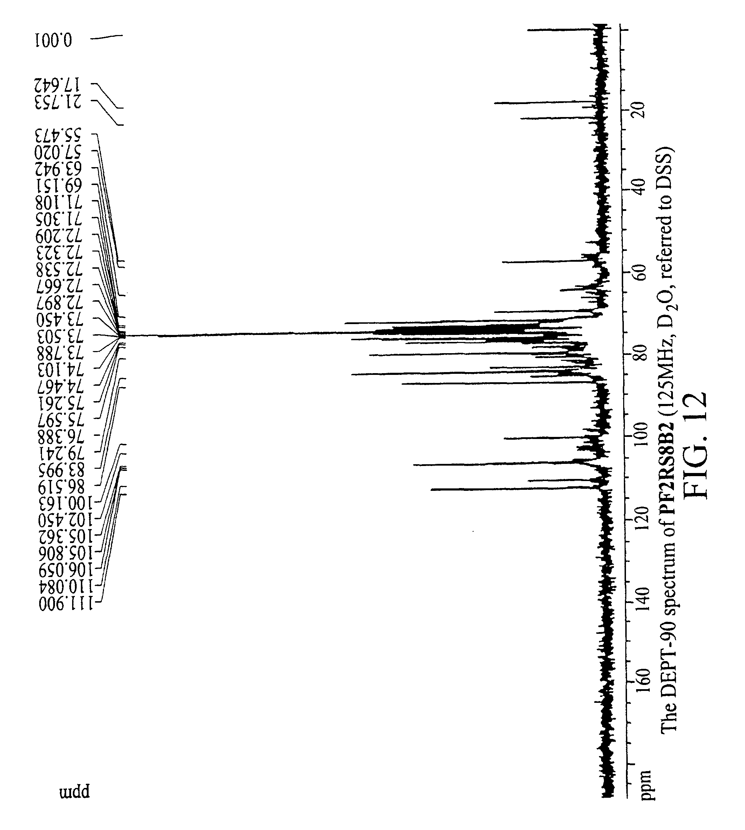

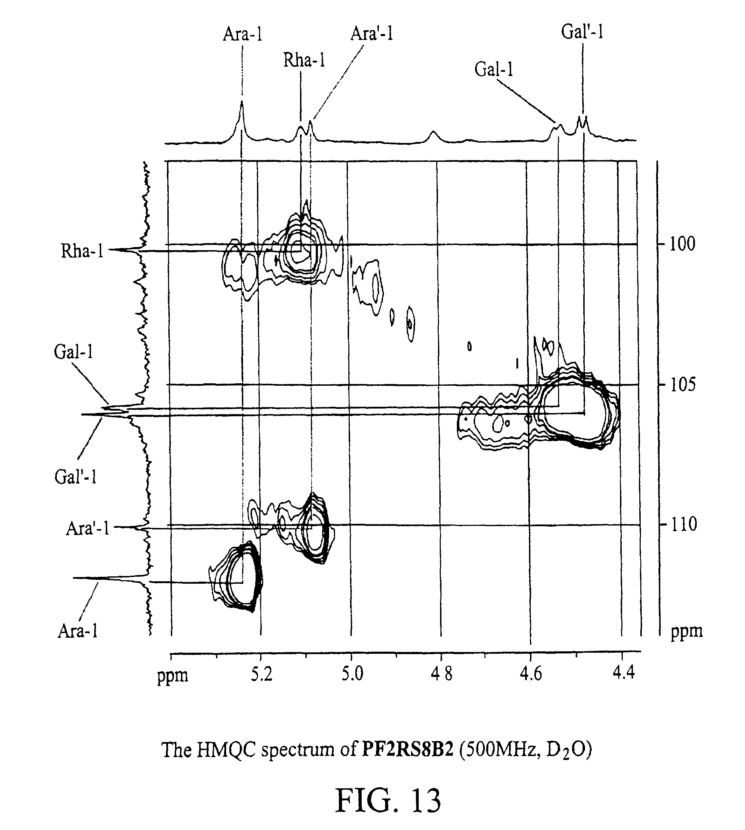

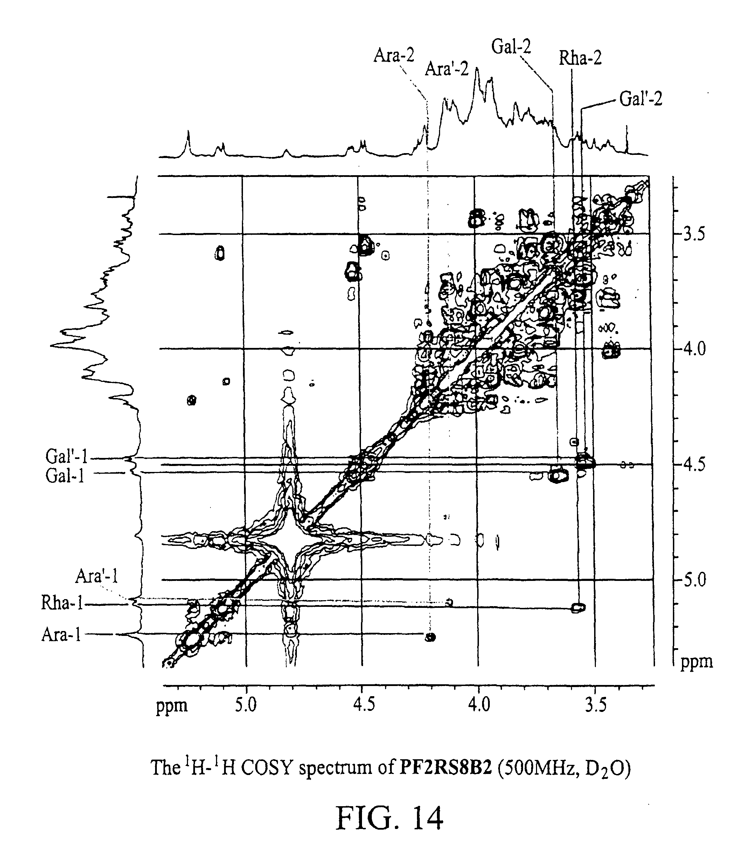

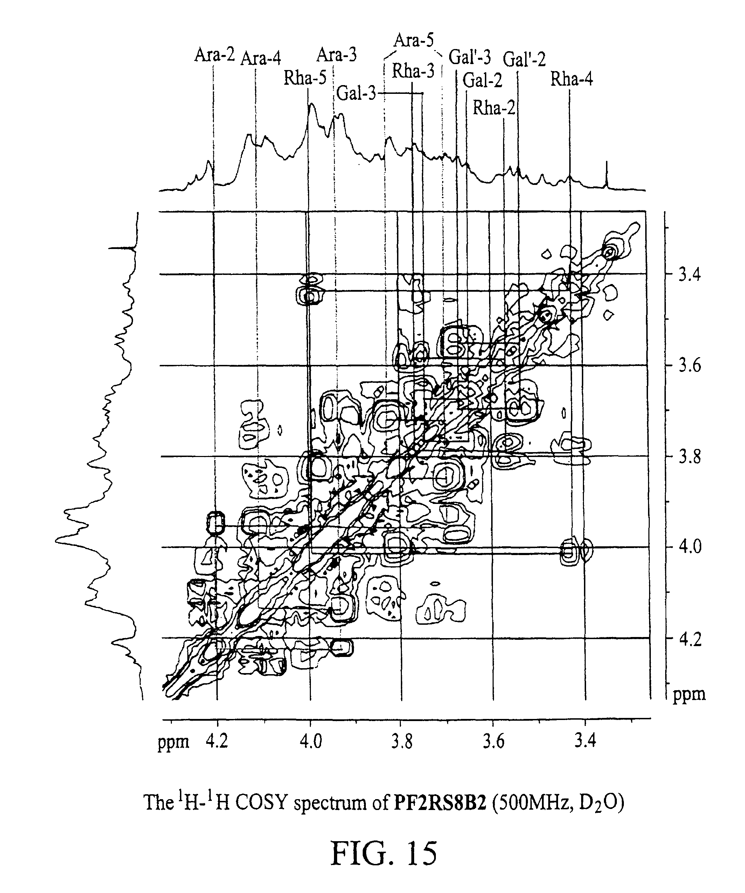

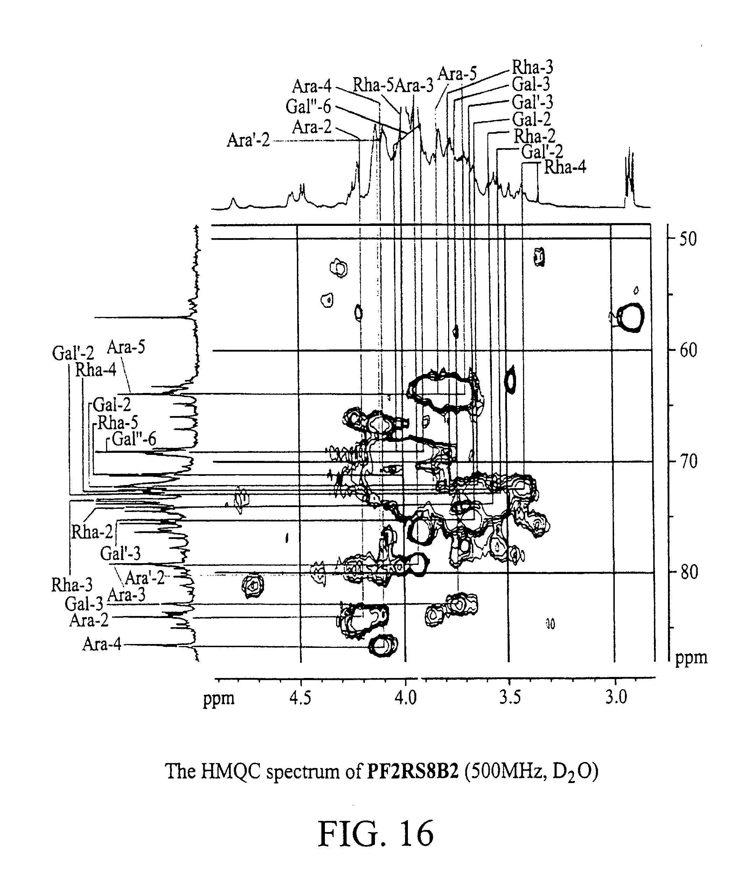

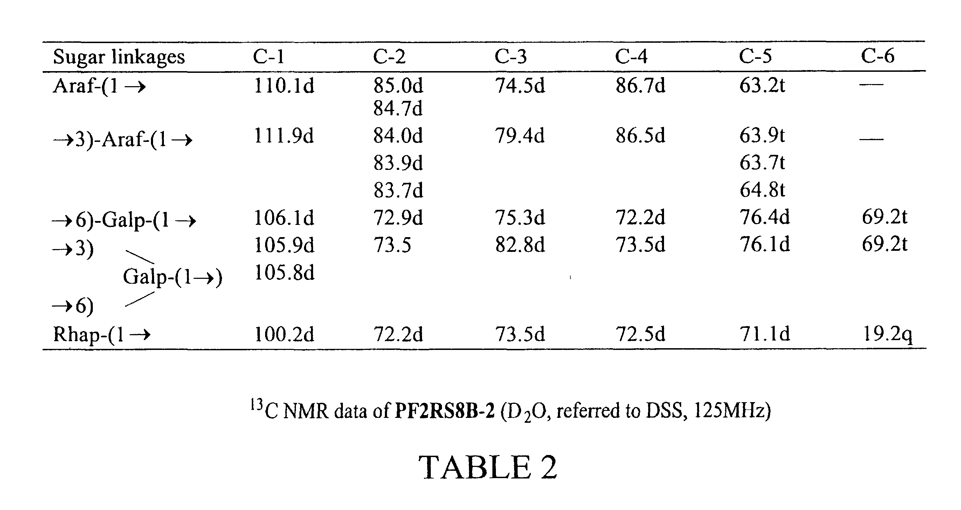

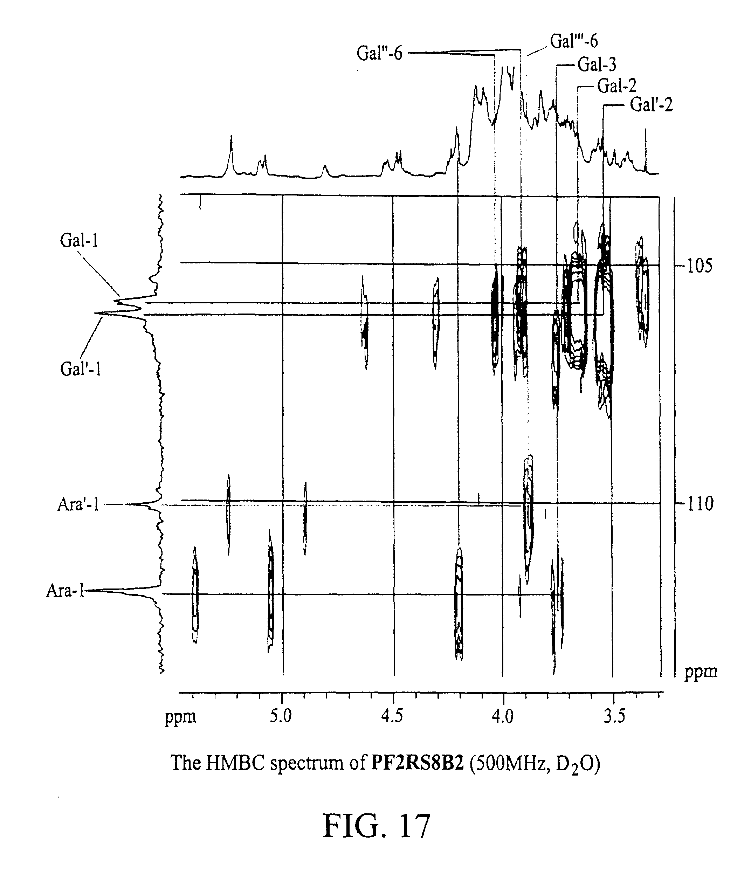

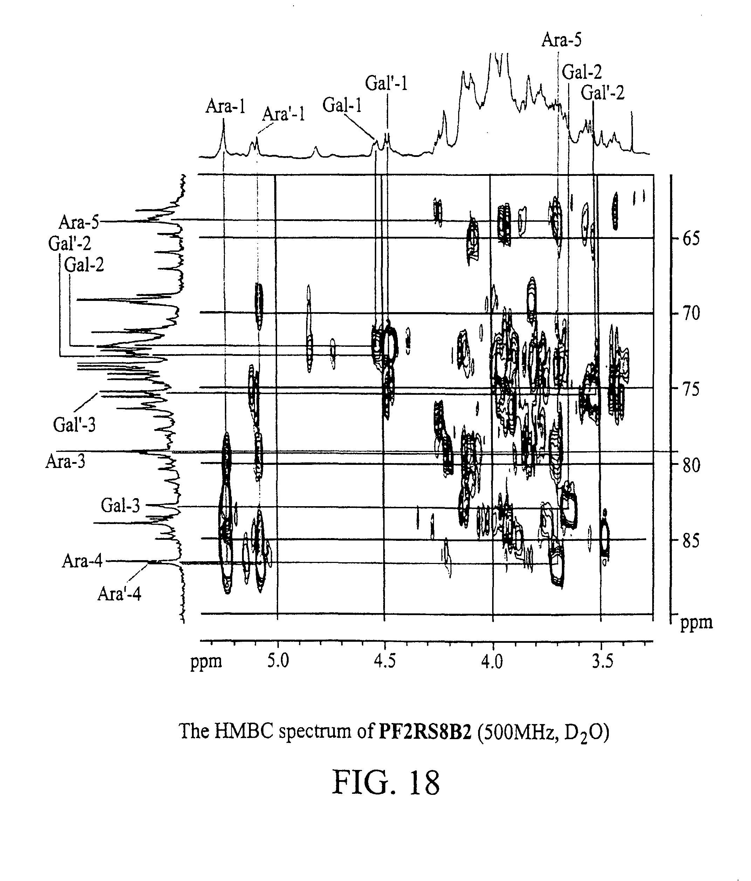

The active polysaccharide **PF2RS8B2** (polysaccharide of formula I) was elucidated by spectroscopic (¹H, ¹³C-NMR, HMQC and 2D ¹H-¹H COSY spectra) and chemical analysis (hydrolysis with TFA and analysis on silica gel TLC). Thus, The ¹H-RMN signals (Figure 9) at δ 5.3 and 3.2 ppm is indicative of a polysaccharide. The ¹³C-NMR signals (Figures 10-12) at δ 111.9 (d), 110.1 (d) ppm were assigned to the anomeric carbons of 1→3 linked α-L-arabinofuranose and terminal α-L-arabinofuranose (expressed Araf and Araf' respectively). The signals at δ106.1 (d) and 105.9 (d) ppm were assigned to the anomeric carbons of 1→6 linked β-D-galactopyranose and 1→3, 1→6 linked β-D-galactopyranose (expressed as Galp' and Galp respectively), whereas, the signal at δ 100.2 (d) ppm was assigned to the anomeric carbons of α-L-rhamnopyranose (expressed as Rhap). The anomeric proton signals (H-1) were also easily recognised due to their relative low-field shift in the ¹H-RMN spectra. The direct correlation between proton and carbon-13 signals observed in HMQC spectrum (Figure 13) determined the ¹H-RMN signals of δ 5.08 (brs), 5.23 (brs), 4,47 (d, J=7.9 Hz), 4.53 (d, J=7.3Hz) and 5.1 (brs) ppm to be the anomeric protons of α-L-arabinofuranose (Araf'), α-L-arabinofuranose (Araf), β-D-galactopyranose (Galp'), β-D-galactopyranose (Galp) and α-L-rhamnopyranose (Rhap). Employing these signals as reference, other proton signals could be traced by analysing 2D ¹H-¹H COSY spectra (figures 14,15). Similarly, the corresponding carbon signals were defined by HMQC spectra (figures 13, 16 and Table 2). The sequencing of the sugar units was determined as follows. The down field shift of the signal at C-3 of Araf (δ 79.4) and Galp (δ 82.8), and the signals at C-6 of Galp and Galp'(δ69.2) suggested that these carbons were connected to other sugar units. The observation of long-range correlations between C-1 of Araf and C-6 of Galp and Galp' in the HMBC spectrum (Figure 17) suggested a 1→6 linked β-D-galactopyranose backbone. The Araf was 1→3 linked to Galp by the observation of long-range correlation between C-1 of Araf and C-3 of Galp (Figure 18). From the ¹H-RMN spectrum (Figure 9), the ratio of the sugars was calculated according to the integration values of anomeric protons peaks to be Araf:Araf':Galp:Galp':Rhap/3:1:2:2:1. PF2RS8B2 was therefore elucidated as a large branched chain polysaccharide with the primary structure as shown in formula I (scheme I). Scheme II is a representation of the same structure showing the stereochemical configuration of the individual sugars.

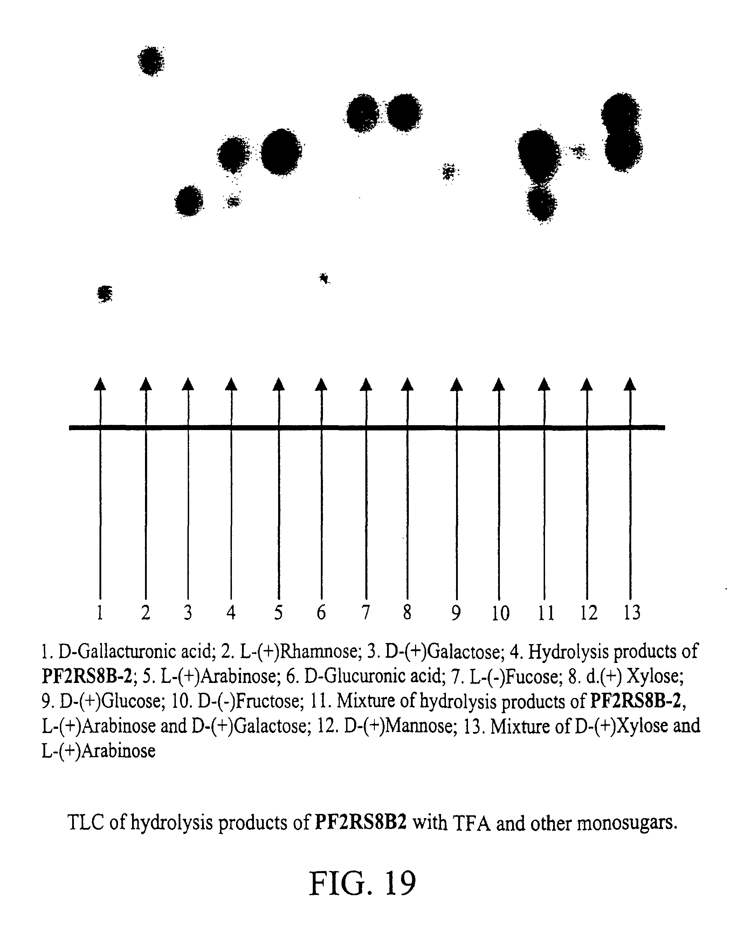

To confirm the identities of the constituent sugars, PF2RS8B2 was hydrolysed with TFA (0.5 M, 100-120 °C) followed by TLC analysis (Figure 19). The presence of the major sugars α-L-arabinofuranose and β-D-galactopyranose was easily discerned, whereas the presence of the minor sugar, α-L-rhamnopyranose, was detected with more difficulties, probably due to the quantity of the hydrolysate applied to the TLC plate.

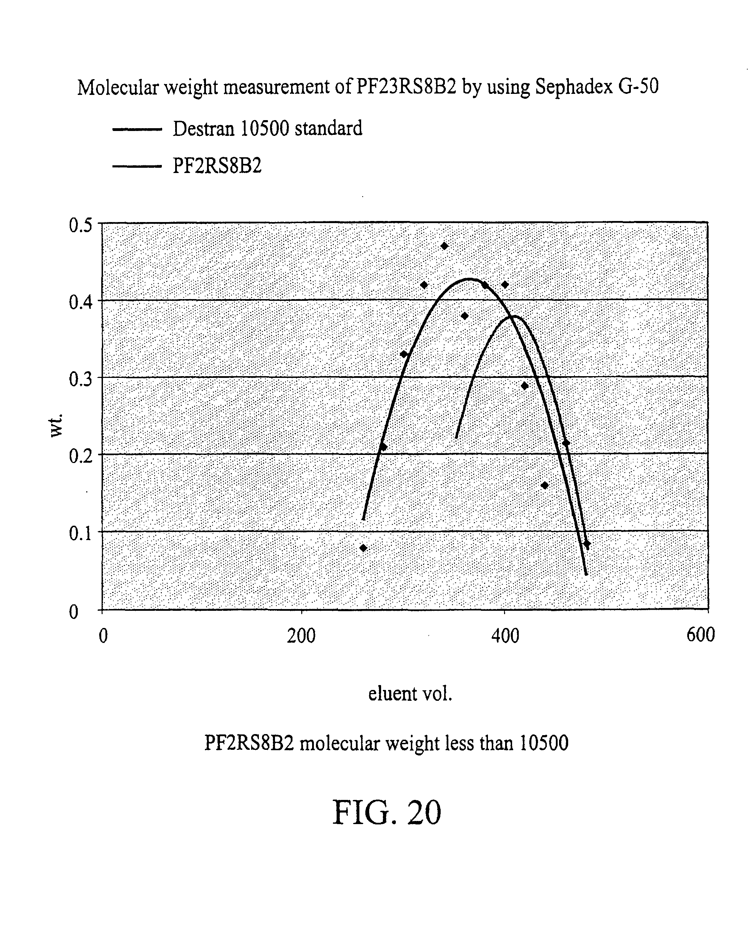

The molecular weight of the **PF2RS8B2** was estimated by gel permeation (size exclusion) chromatography. The mean molecular weight thus estimated of PF2RS8B2 is of 10,000 (See figure 20).

The polysaccharide compound of formula I unexpectedly presents very high activity as immune stimulator, as reflected in the example below. The third aspect of the invention relates, thus, to the use of the polysaccharide compound of formula I as therapeutic agent in the treatment of immune-suppressant diseases such as cancer, tuberculosis, influenza, common cold, allergies, lupus erythematosus, psoriasis and AIDS. Non limitative examples of cancers are hepatic carcinoma, lung cancer, kidney cancer, colon cancer, breast cancer, prostate cancer or prostatic adenocarcinoma; brain cancers such as astrocytoma and glioblastoma; cervix cancer and blade cancer.

The fourth aspect of the invention relates to pharmaceutical compositions comprising the polysaccharide compound of formula I.

The polysaccharide according to the present invention can be administrated either separately as a pure substance or in the form of pharmaceutical preparations, even though the compound of the invention is preferably administered in a combination. The drug combination is preferably in the form of a formulation which (1) contains the polysaccharide according to the invention alone; (2) contains one or more appropriate binders, carriers and/or further auxiliary materials, and (3) may further contain additional therapeutically active substances.

The carriers materials, binders and/or auxiliary materials must be pharmaceutically and pharmacologically tolerable, so that they can be combined with the other components of the formulation or preparation and do not exert adverse effects on the organism treated.

The formulations include those which are suitable for oral or parentheral (including subcutaneous, intradermal, intramuscular and intravenous) administration, even though the best route of administration is dependent on the patient's status.

The formulations can be in the form of single doses. The formulations are prepared according to methods known in the field of pharmacology. The appropriate quantities of active substances suitable for administration may vary as a function of the particularly field therapy. In general, the active substance concentration in a single-dose formulation is 5% to 95% of the total formulation.

The invention provided by the application is illustrated by the examples presented hereinbelow.
**Example 1:** Preparation of an aqueous extract of flowers of *Calendula oficinalis* according to the method of the invention.
   500 g. of flowers of *Calendula oficinalis* are placed in a wash tunnel and subjected to a thorough wash with water at about 28°C. The flowers are next comminuted with a comminuting machine. The resultant 500 g of comminuted matter are subjected to a treatment with a red linear laser diode with a capability of harmonic generation in a wavelength of 250 nm, a power of 20 watts and a spot of 4 mm of diameter. The treatment is effected by manually displacing the laser generator through the comminuted matter during 2.5 minutes, so that the whole or most of the mixture is irradiated. The laser treated matter is next suspended in 2 litres of water at a temperature of about 20 °C. The suspension is then kept for 12 days at a temperature of 4 °C. Finally, the separation of the liquid and the solid phase is effected, first by decantation of the liquid (the solids are pressed to facilitate the separation), and then, by three consecutive press-filtrations with filters of 5, 1 and 0.22 µm at a temperature of about 20 °C. The process yields approximately 1.7 litres of a solution (aqueous extract) of an ochre colour.
**Example 2:** The isolation of the polysaccharide of formula I is effected as disclosed on pages 7-8 of the description.
**Example 3:** The polysaccharide of formula I was tested in order to establish its activity as immune stimulator by quantifying the lymphocyte transformation activity (LTA). By lymphocyte transformation activity it is meant the fact that the lymphocytes are transformed from a dormant to an active state, which is necessary to fight diseases through an immunological mechanism, or to restore the immune system, which might be weakened by different factors. The tests were performed according to the literature reference *Max, W. et al., Journal of Natural Products, vol. 54, no. 6, pp. 1531-1542 (1991)*, by adding in vitro a solution of the polysaccharide of the invention to lymphocytes isolated from mice. The incorporation of thymidine, which means replication of DNA, was monitored. This incorporation is indicative both of an increase in lymphocyte number and an increase in lymphocyte activity.
   The polysaccharide of formula I presents a LTA of +6203 % with respect to non stimulated lymphocytes.

## Claims

1. Polysaccharide according to the formula I , 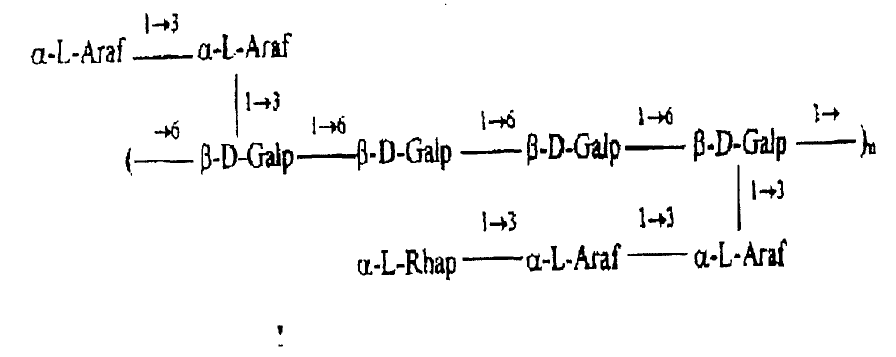 wherein n = 7 to 8.

2. Method for obtaining the compound defined in claim 1 which comprises 1) separating the flowers of the plant *Calendula officinalis*, 2) subjecting them the following extraction method:
a) Decontamination of the flowers
b) Comminuting the flowers.
c) Treatment of the comminuted flowers with a laser radiation.
d) Suspension of the mixture obtained in step c) in water.
e) Maceration of the suspension obtained in step d).
f) Separation of the resulting liquid.
and 3) subjecting the liquid obtained in step f) to an isolation process which comprises precipitation with methanol, centrifugation and chromatographic separation guided by bioassay.

3. Method according to claim 2 in which the step 3) comprises:
g) Lyophilisation of the liquid obtained in step f).
h) Precipitation of the lyophilised matter obtained in step g) with methanol.
i) Separation of the solid phase from the liquid phase.
j) Precipitating the solid phase obtained in step i) with methanol to final concentrations of 25%, 50% and 67%.
k) Solubilising in water the precipitates obtained in step j) at 50% and 67%, centrifugating and effecting a chromatographic separation of the supernatant.
l) Identifying the active fraction by bioassay.
m) Effecting a second chromatographic separation of the active fraction.
n) Identifying the active fraction by bioassay.
o) elimination of the eluent.

4. Method according to claims 2 and 3, wherein the treatment with the laser radiation is effected with a red linear laser diode with a capability of harmonic generation in wavelengths within the range of 150 to 810 nm, a power of 1 to 60 watts and a spot of 1 to 6 mm of diameter.

5. Method according to claim 4, wherein the wavelength is within the range of 200 to 400 nm, preferably 250 nm, the power of 20 watts and the spot of 4 mm of diameter.

6. Method according to any precedent claim, wherein each Kilogram of the comminuted matter is treated with the laser radiation for a period of 3 to 10 minutes, preferably for a period of 5 minutes.

7. Compound defined in claim 1 for use as therapeutic agent.

8. Compound according to claim 1 for use in the treatment of immune suppressant diseases.

9. Compound according to claim 1 for use in the treatment of immune-suppressant diseases such as cancer, tuberculosis, influenza, common cold, allergies, lupus erythematosus, psoriasis and AIDS.

10. Compound according to claim 1 for use in the treatment of hepatic carcinoma, lung cancer, kidney cancer, colon cancer, breast cancer, prostate cancer or prostatic adenocarcinoma; brain cancers such as strocytoma and glioblastoma; cervix cancer and blade cancer.

11. Use of the compound defined in claim 1 for the manufacture of a medicament.

12. Use according to claim 11 for the manufacture of a medicament for treating immune suppressant diseases.

13. Use according to claim 12 for the manufacture of a medicament for treating cancer, tuberculosis, influenza, common cold, allergies, lupus erythematosus, psoriasis and AIDS.

14. Use according to claim 13 for the manufacture of a medicament for treating hepatic carcinoma, lung cancer, kidney cancer, colon cancer, breast cancer, prostate cancer or prostatic adenocarcinoma; brain cancers such as strocytoma and glioblastoma; cervix cancer and blade cancer.

15. A pharmaceutical preparation comprising a compound according to claim 1.

16. The pharmaceutical preparation according to claim 15 further including a pharmaceutically an acceptable vehicle.

17. The pharmaceutical preparation of claims 15 and 16 further including at least another pharmaceutically active compound.

## Patentansprüche

1. Polysaccharid der Formel I, 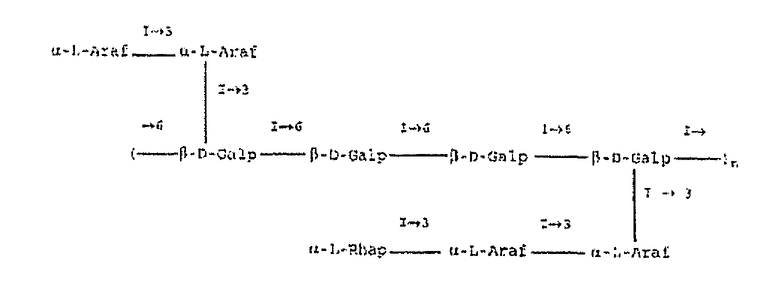 worin n = 7 bis 8 ist.

2. Verfahren zum Erhalt der Verbindung nach Anspruch 1, umfassend (1) das Abtrennen der Blüten der Pflanze *Calendula officinalis*, (2) Behandlung derselben mit dem folgenden Extraktionsverfahren
a) Entgiften der Blüten,
b) Zerkleinern der Blüten,
c) Behandeln der zerkleinerten Blüten mit Laserstrahlung,
d) Suspendieren des in Schritt c) erhaltenen Gemischs in Wasser,
e) Mazerieren der in Schritt d) erhaltenen Suspension,
f) Abtrennen der erhaltenen Flüssigkeit und (3) Unterziehen der in Schritt f) erhaltenen Flüssigkeit einem Isollerungsverfahren, umfassend die Ausfällung mit Methanol, Zentrifugation und durch einen Bioassay durchgeführte chromatografische Auftrennung.

3. Verfahren nach Anspruch 1, in welchem Schritt (3) Folgendes umfasst:
g) Lyophilisation der in Schritt f) erhaltenen Flüssigkeit,
h) Fällen der in Schritt g) erhaltenen lyophilisierten Substanz mit Methanol,
i) Abtrennen der festen Phase von der flüssigen Phase,
j) Fällen der in Schritt i) erhaltenen festen Phase mit Methanol auf Endkonzentrationen von 25 %, 50 % und 67 %,
k) Anlösen der in Schritt j) erhaltenen Niederschläge mit 50 % und 67 %, Zentrifugieren einer chromatografischen Auftrennung des Überstands,
l) Identifizieren der aktiven Fraktion durch einen Bioassay,
m) Zweiten chromatografischen Auftrennung der aktiven Fraktion,
n) Identifizieren der aktiven Fraktion durch einen Bioassay,
o) Entfernen des Elutionsmittels.

4. Verfahren nach den Ansprüchen 2 und 3, worin Laserbestrahlung mit einer roten linearen Laserdiode mit der Fähigkeit der Frequenzvervielfachung in Wellenlängen im Bereich von 150 bis 180 nm, einer Leistung von 1 bis 60 Watt und einem Leuchtpunkt mit einem Durchmesser von 1 bis 6 mm bewirkt wird.

5. Verfahren nach Anspruch 4, worin die Wellenlänge im Bereich von 200 bis 400 nm liegt, vorzugsweise 250 nm beträgt, die Leistung 20 Watt beträgt und der Leuchtpunkt einen Durchmesser von 4 mm aufweist.

6. Verfahren nach Anspruch 4, wobei jedes Kilogramm der zerkleinerten Substanz bis 10 Minuten lang, vorzugsweise 5 Minuten lang mit der Laserstrahlung behandelt wird.

7. Verbindung, nach Anspruch 1, zur Verwendung als therapeutisches Mittel.

8. Verbindung nach Anspruch 1 zur Verwendung bei der Behandlung von immununterdrückenden Erkrankungen.

9. Verbindung nach Anspruch 1 zur Verwendung bei der Behandlung von immununterdrückenden Erkrankungen wie Krebs, Tuberkulose, Influenza, Erkältung, Allergien, Lupuserythematodes, Psoriasis und AIDS.

10. Verbindung nach Anspruch 1 zur Verwendung bei der Behandlung von Leberkarzinomen, Lungenkrebs, Nierenkrebs, Darmkrebs, Brustkrebs, Prostatakrebs oder Prostataadenokarzinomen, Gehimkrebsen wie Strocytomen und Glioblastomen, Gebärmutterhalskrebs und Blasenkrebs.

11. Verwendung der Verbindung nach Anspruch 1 definierten Verbindung zur Herstellung eines Medikaments.

12. Verwendung nach Anspruch 11 zur Herstellung eines Medikaments zur Behandlung von immununterdrückenden Erkrankungen.

13. Verwendung nach Anspruch 12 zur Herstellung eines Medikaments zur Behandlung von immununterdrückenden Erkrankungen wie Krebs, Tuberkulose, Influenza, Erkältung, Allergien, Lupuserythematodes, Psoriasis und AIDS.

14. Verwendung nach Anspruch 13 zur Herstellung eines Medikaments zur Medikaments zur Behandlung von Leberkarzinomen, Lungenkrebs, Nierenkrebs, Darmkrebs, Brustkrebs, Prostatakrebs oder Prostataadenokarzinomen, Gehirnkrebsen wie Strocytomen und Glioblastomen, Gebärmutterhalskrebs und Blasenkrebs.

15. Pharmazeutisches Präparat, umfassend eine Verbindung nach Anspruch 1.

16. Pharmazeutisches Präparat nach Anspruch 15, des Weiteren einschließend einen pharmazeutisch verträglichen Träger.

17. Pharmazeutisches Präparat nach den Ansprüchen 15 und 16, des Weiteren einschließend mindestens einen anderen pharmazeutischen Wirkstoff.

## Revendications

1. Polysaccharide selon la formule I : 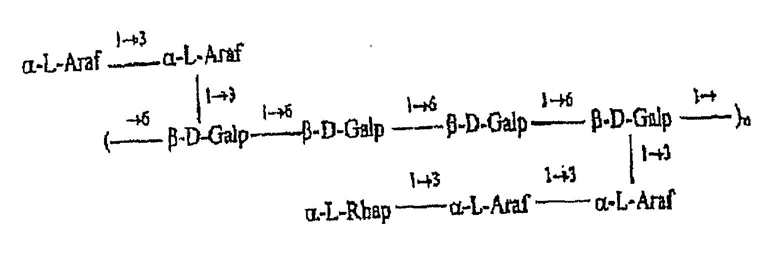 dans laquelle n = 7 à 8.

2. Procédé pour obtenir le composé selon la revendication 1, **caractérisé en ce que** les étapes suivantes : 1) séparer les fleurs de la plante *Calendula officinalis*, 2) les soumettre au procédé d'extraction suivant qui comprend :
a) la décontamination des fleurs,
b) le broyage des fleurs,
c) le traitement des fleurs broyées avec un rayonnement laser,
d) la mise en suspension dans de l'eau du mélange obtenu à l'étape c),
e) la macération de la suspension obtenue à l'étape d),
f) la séparation du liquide obtenu,
et 3) soumettre le liquide obtenu à l'étape f) à un procédé d'isolement qui comprend la précipitation avec du méthanol, la centrifugation et la séparation chromatographique guidée par dosage biologique.

3. Procédé selon la revendication 2, **caractérisé en ce que** l'étape 3) comprend :
g) la lyophilisation du liquide obtenu à l'étape f).
h) la précipitation de la matière lyophilisée obtenue à l'étape g) avec du méthanol,
i) la séparation de la phase solide et de la phase liquide,
j) la précipitation de la phase solide obtenue à l'étape i) avec du méthanol pour obtenir des concentrations finales de 25 %, 50 % et 67 %,
k) la solubilisation dans l'eau des précipités obtenus à l'étape j) à 50 % et 67 %, la centrifugation et la séparation chromatographique du surnageant,
l) l'identification de la fraction active par dosage biologique,
m) la seconde séparation chromatographique de la fraction active,
n) l'identification de la fraction active par dosage biologique,
o) l'élimination de l'éluant.

4. Procédé selon les revendications 2 et 3, **caractérisé en ce que** le traitement avec le rayonnement laser est effectué avec une diode laser linéaire rouge capable de générer des harmoniques d'une longueur d'onde allant de 150 à 810 nm, d'une puissance de 1 à 60 watts et ayant un faisceau de 1 à 6 mm de diamètre.

5. Procédé selon la revendication 4, **caractérisé en ce que** la longueur d'onde va de 200 à 400 nm, est de préférence de 250 nm, dont la puissance est de 20 watts et le faisceau fait 4 mm de diamètre.

6. Procédé selon l'une quelconque des revendications précédentes, **caractérisé en ce que** chaque kilogramme de matière broyée est traité avec un rayonnement laser sur une période de 3 à 10 minutes, de préférence sur une période de 5 minutes.

7. Composé selon la revendication 1, utilisé comme agent thérapeutique.

8. Composé selon la revendication 1, utilisé dans le traitement de maladies immunosuppressives comme le cancer, la tuberculose, le coryza, les allergies, le lupus erthémateux, le psoriasis et le SIDA.

9. Composé selon la revendication 1, utilisé dans le traitement de maladies immunosuppressives comme le cancer, la tuberculose, la grippe, le coryza, les allergies, le lupus érythémateux, le psoriasis et le SIDA.

10. Composé selon la revendication 1, utilisé dans le traitement du carcinome hépatique, du cancer du poumon, du cancer du rein, du cancer du côlon, du cancer du sein, du cancer de la prostate ou de l'adénocarcinome prostatique, des cancers du cerveau comme l'astrocytome et le glioblastome, du cancer du col de l'utérus et du cancer de la vessie.

11. Utilisation du composé selon la revendication 1, pour fabriquer un médicament.

12. Utilisation selon la revendication 11, pour la fabrication d'un médicament destiné à traiter des maladies immunosuppressives.

13. Utilisation selon la revendication 12, pour la fabrication d'un médicament destiné à traiter le cancer, la tuberculose, la grippe, le coryza, les allergies, le lupus érythémateux, le psoriasis et le SIDA.

14. Utilisation selon la revendication 13, pour la fabrication d'un médicament destiné à traiter le carcinome hépatique, le cancer du poumon, le cancer du rein, le cancer du côlon, le cancer du sein, le cancer de la prostate ou l'adénocarcinome prostatique, les cancers du cerveau comme l'astrocytome et le glioblastome, le cancer du col de l'utérus et le cancer de la vessie.

15. Préparation pharmaceutique comprenant un composé selon la revendication 1.

16. Préparation pharmaceutique selon la revendication 15, comprenant en outre un véhicule pharmaceutiquement acceptable.

17. Préparation pharmaceutique selon les revendications 15 et 16, comprenant en outre au moins un autre composé pharmaceutiquement actif.
